# EUROPEAN PATENT SPECIFICATION

(11) **EP 2 866 651 B1**
(45) Date of publication and mention of the grant of the patent: **01.11.2017**
(21) Application number: 13809703.5
(22) Date of filing: 28.06.2013
(51) Int. Cl.: A61B 5/103, A61F 2/28, G06F 19/00, G01C 17/38, A61B 17/56

(54) **AN ELECTRONIC ORIENTATION MONITOR**
MONITOR MIT ELEKTRONISCHER AUSRICHTUNG
MONITEUR D'ORIENTATION ÉLECTRONIQUE

(30) Priority: 28.06.2012 AU 2012902752; 12.04.2013 AU 2013204920
(43) Date of publication of application: 06.05.2015
(73) Proprietor: Gyder Surgical Pty Ltd, Sydney, NSW 2000 (AU)
(72) Inventor: LYE, Robert, Brookvale, New South Wales 2100 (AU)
(74) Representative: Cabinet Plasseraud
(86) International application number: PCT/AU2013/000713
(87) International publication number: WO 2014/000053

(56) References cited:
- WO-A1-02/080824
- WO-A2-2004/112610
- DE-U1-202009 013 604
- US-A1- 2009 254 294
- US-A1- 2010 076 446
- US-A1- 2011 208 093
- US-A1- 2011 208 093
- US-A1- 2012 130 279

## Description

### Field of the Invention

The present invention relates to surgical implements and in particular to an electronic orientation monitor that may be used prior to and during surgical procedures, for example surgery involving prosthetic components.

### Background

The discussion of the prior art within this specification is not, and should not be taken as, an admission of the extent of common general knowledge in the field of the invention. Rather, the discussion of the prior art is provided merely to assist the addressee to understand the invention and is included without prejudice.

Whilst the following discussion is with respect to hip replacement surgery, a person skilled in the art will appreciate that the present invention is not limited to this particular field of use and may be adapted to use with any bone structure or various types of surgery.

Hip replacement surgery involves the use of a prosthetic cup (acetabular cup) or a prosthetic ball (femoral stems) or both to restore the ball and cup joint functionality of the hip. The ball and cup joint enables the hip to rotate in different directions to various degrees (in contrast to the relatively limited rotation of a knee joint).

Historically, hip replacement (arthroplasty) surgery required up to a 40cm (7 to 12 inches) curved incision to provide sufficient access for the surgeon to manually access and manipulate the hip and femur. A prosthetic cup was attached to the hip socket or the head of the femur removed and replaced with a prosthetic ball, or both.

After the incision is made, the ligaments and muscles are separated to allow the surgeon access to the bones of the hip joint. It is typically this part of the surgery that makes the ligaments and muscles somewhat weak after surgery. Until they heal, which often takes about a month to six weeks, the patient must follow special hip precautions to prevent dislocation of the new hip joint.
Typical steps in hip replacement surgery include the following:
- *Removing the Femoral Head:* Once the hip joint is entered, the femoral head is dislocated from the acetabulum. Then the femoral head is removed by cutting through the femoral neck with a power saw.
- *Reaming the Acetabulum:* After the femoral head is removed, the cartilage is removed from the acetabulum using a power drill and a special reamer. The reamer forms the bone in a hemispherical shape to exactly fit the metal shell of the acetabular component.
- *Inserting the Acetabular Component:* A trial component, which is an exact duplicate of the patient's hip prosthesis, is used to ensure that the joint received will be the right size and fit. Once the right size and shape is determined for the acetabulum, the acetabular component is inserted into place. In the uncemented variety of artificial hip replacement, the metal shell is simply held in place by the tightness of the fit or with screws to hold the metal shell in place. In the cemented variety, a special epoxy type cement is used to "glue" the acetabular component to the bone.
- *Preparing the Femoral Canal:* To begin replacing the femoral head, special rasps are used to shape and hollow out the femur to the exact shape of the metal stem of the femoral component. Once again, a trial component is used to ensure the correct size and shape. The surgeon will also test the movement of the hip joint.
- *Inserting the Femoral Stem:* Once the size and shape of the canal exactly fit the femoral component, the stem is inserted into the femoral canal. Again, in the uncemented variety of femoral component the stem is held in place by the tightness of the fit into the bone (similar to the friction that holds a nail driven into a hole drilled into wooden board with a slightly smaller diameter than the nail). In the cemented variety, the femoral canal is rasped to a size slightly larger than the femoral stem, then the epoxy type cement is used to bond the metal stein to the bone.
- *Attaching the Femoral Head:* The metal ball that replaces the femoral head is attached to the femoral stem. The Completed Hip Replacement: Before the incision is closed, an x-ray is taken to make sure the new prosthesis is in the correct position.

Such surgery had a number of problems including:
▪ a hospital stay of three days or more, post-operative pain and weeks of rehabilitation;
▪ each cm of incision has a tenfold increase in the risks of blood clotting and
▪ infection post surgery; and
▪ the surgeon was reliant on his experience and eye to ensure accurate placement of the cup into the three dimensional hip socket and alignment of the cup with the ball/femur to enable proper function of the joint. Misalignment may lead to post operative complication such as misalignment of the leg, incorrect leg length and/or incorrect soft tissue tension. The long term effects of misaligned prosthetic components can also include accelerated wear of the components, aseptic loosening of the components and potentially early repetition of the surgery.

Attempts to overcome these problems include:
▪ WO 2003/037192 which discloses a jig (impaction tool) for use in bone surgery and thus enables the use of a smaller incision. For hip replacement surgery, the jig enables the use of a 4 to 7 cm (2 to 3 inch) incision, i.e. keyhole surgery. Other benefits include a shorter stay in hospital, less blood loss, less pain, fewer postoperative dislocations and faster recovery; and
▪ WO 2005/046475 which discloses a gauge a gauge to assist the surgeon with accurate placement of a prosthetic when using a jig in keyhole surgery as the surgeon is no longer able to see the fit of the cup into the hip socket or the fit between the ball and cup.

The gauge provided in WO 2005/046475 has enabled efficient use of the impaction tool of WO 2003/037192. Commercial examples include the NilNav Hip System available from MAC Surgical. However, the gauge only works in two dimensions and there is still a heavy reliance on the surgeon's eye and experience for optimal placement of the cup into the hip.

A further attempt to overcome these problems was provided by WO 2010/031111, the contents of which are hereby incorporated in their entirety into this specification by way of cross reference. This prior art document discloses a brace (3) in the form of a clamp 20 that is attachable to a patient to define a reference point relative to the patient's anatomy for calibration of an electronic orientation monitor (2). It also discloses subsequent indications provided by a LED array (26) of the electronic orientation monitor (2), which may be used to assist in manipulation of a surgical implement (1). However, it has been appreciated by the present inventor that the information displayed by the LED array (26) of this prior art electronic orientation monitor (2) is limited in its extent and user friendliness.

An electronic orientation monitor according to the preamble of claim 1 is disclosed in US 2011/208093.

Further examples of electronic orientation monitors are disclosed in WO 2004/112610, WO 02/080824 and DE 20 2009 013604.

### Summary of the Invention

In one aspect of the present invention there is provided an electronic orientation monitor including:
orientation sensing electronics configured for calibration when in a reference orientation and being responsive to manipulation of the monitor so as to calculate first, second and third angles which together represent a difference between a current orientation of the monitor and the reference orientation; and
a display being responsive to the first and second angles so as to display a point positioned relative to first and second axes, the display being further responsive to the third angle so as to display a line having a direction relative to the first and second axes such that a combination of the position of the point and the direction of the line is indicative to a user of the difference between the current orientation of the monitor and the reference orientation.

In one embodiment the first, second and third angles are respectively associated with a three dimensional reference system that is defined with reference to a roll angle, a pitch angle and a yaw angle. In this embodiment the monitor is configured during calibration to sense and store a reference roll angle, a reference pitch angle and a reference yaw angle.

Preferably the monitor is configured to calculate the first angle by sensing a current roll angle and comparing the reference roll angle to the current roll angle and it is configured to calculate the second angle by sensing a current pitch angle and comparing the reference pitch angle to the current pitch angle and it is configured to calculate the third angle by sensing a current yaw angle and comparing the reference yaw angle to the current yaw angle.

A coordinate of the position of the point on the first axis is determined with reference to the first angle and a coordinate of the position of the point on the second axis is determined with reference to the second angle. The line extends from an origin of the first and second axes.

Preferably a direction of the line is determined with reference to the third angle.

A correspondence between the current orientation and the reference orientation is indicated on the display by the point being disposed on an origin of the first and second axes and the line being aligned with a reference indicium.

Preferably the reference indicium is a predetermined one of the first or second axes. In this embodiment the point is indicated on the display as the point of intersection of two lines and the point is also indicated on the display by the centre of a circle. In this embodiment the display includes a numeric display of the first, second and third angles.

A method of guiding manipulation of an implement using an electronic orientation monitor as described above is also disclosed. The method includes the steps of:
calibrating the electronic orientation monitor when in a reference orientation;
attaching the electronic orientation monitor to the implement; and
manipulating the implement until the circle on the display of the electronic orientation monitor is positioned substantially on the origin of the first and second axes and the line on the display of the electronic orientation monitor is substantially aligned with the reference indicium so as to indicate that a current orientation of the monitor corresponds to the reference orientation.

The features and advantages of the present invention will become further apparent from the following detailed description of preferred embodiments, provided by way of example only, together with the accompanying drawings.

### Brief Description of the Accompanying Drawings

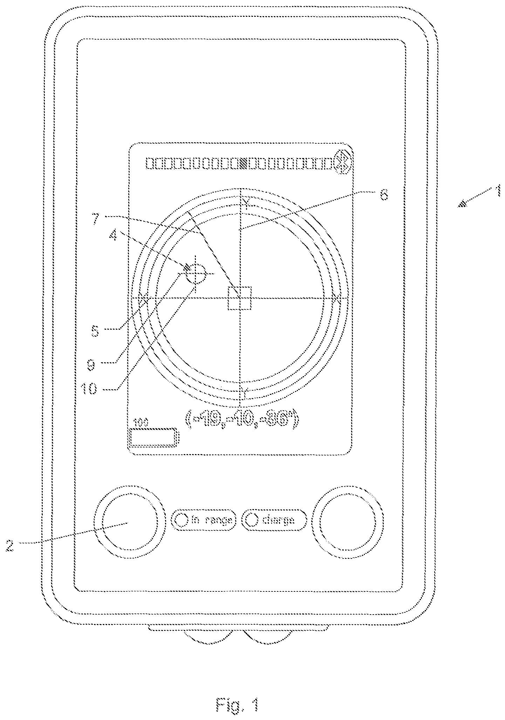

Figure 1 is a plan view of an embodiment of the electronic orientation monitor according to the invention showing a display upon which an orientation that diverges from the reference orientation is depicted;

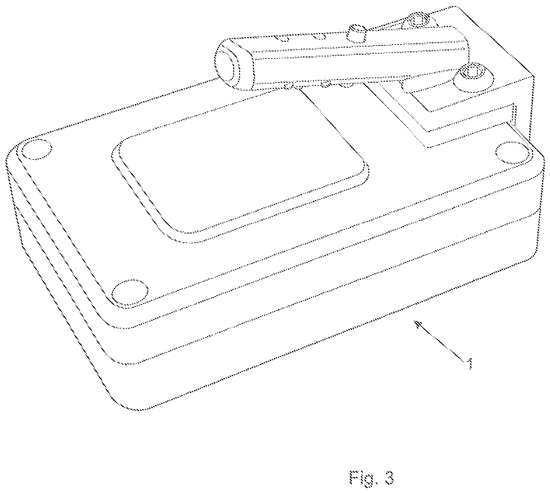

Figure 2 is a plan view of the embodiment of Figure 1 showing a display upon which an orientation that corresponds to the reference orientation is depicted; and Figure 3 is a bottom side perspective view of the embodiment of Figure 1.

### Detailed Description of Preferred Embodiments of the Invention

The electronic orientation monitor 1 includes orientation sensing electronics that are disposed within the casing shown in the figures. The details of an embodiment of the sensing electronics are disclosed in WO 2010/031111, the contents of which have been incorporated in their entirety into this specification by way of cross reference. The sensing electronics are calibrated when in the electronic orientation monitor 1 has been placed in a reference orientation. The brace disclosed in WO 2010/031111, or alternative braces and/or other referencing apparatuses and methods, may be used to place the electronic orientation monitor 1 into the reference orientation. Once in the reference orientation, the user presses the calibration button 2 and the monitor's processor causes the orientation sensing electronics to sense the reference orientation, which is stored in the monitor's random access memory. More particularly, the orientation sensing electronics generate data that is representative of three reference angles, which are respectively associated with a three dimensional reference system comprising a roll angle, a pitch angle and a yaw angle. Hence, upon calibration, the orientation sensing electronics senses data that is representative of a reference roll angle, a reference pitch angle and a reference yaw angle. Each of these angles is a component of the overall reference orientation and hence, together, these three angles define the reference orientation.

Once calibrated, the electronic orientation monitor 1 is typically detached from the referencing apparatus and then rigidly attached to a surgical implement such that the electronic orientation monitor I moves as one with the implement. As the electronic orientation monitor I is manipulated whilst attached to the implement, its orientation sensing electronics continue to generate data that is representative of current values for the roll angle, the pitch angle and the yaw angle. This data is communicated to the monitor's processor, which is programmed to compare the current values to the reference values so as to calculate first, second and third angles. More particularly, the processor subtracts the stored reference roll angle from the current roll angle to calculate the first angle, It subtracts the stored reference pitch angle from the current pitch angle to calculate the second angle. It subtracts the stored reference yaw angle from the current yaw angle to calculate the second angle. Together the first, second and third angles represent a difference between a current orientation of the monitor and the reference orientation.

The monitor's display 3 may take the form of any screen that can be driven by executable software instructions to display graphics. In some preferred embodiments it is a liquid crystal display and in some alternative embodiments it is an organic light-emitting diode display. The display 3 is used to present visual information to the user that is indicative of the first, second and third angles and which may therefore be used to help guide the monitor I into a desired orientation, for example towards the reference orientation. The visual information that is responsive to the first and second angles takes the form of a point 4 positioned relative to a first axis 5 (labeled the 'X' axis in the figures) and a second axis 6 (labeled the 'Y' axis in the figures). The visual information also takes the form of a line 7 that extends from the origin 8 of the first and second axes and 6 in a direction that is dependent upon the third angle. Hence, a combination of the position of the point 4 and the direction of the line 7 is indicative to a user of the difference between the current orientation of the monitor I and the reference orientation.

To assist the user to identify the point 4, it is indicated on the display 3 as the point of intersection of two lines 9 and 10. Additionally, it is indicated on the display as the centre of circle 11.

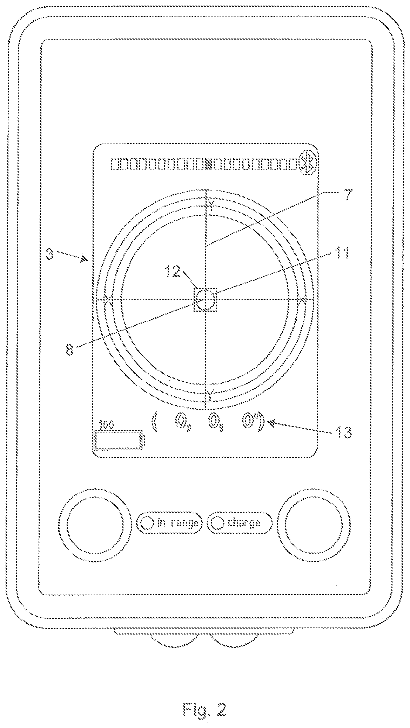

The monitor's processor is programmed with an algorithm or formula that is used to calculate the coordinates of the point 4 based on the first and second angles. In one embodiment, there is a linear relationship between the value of the first angle and the coordinate on the first axis 5 at which the point 4 is displayed. Similarly, in this embodiment there is a linear relationship between the value of the second angle and the coordinate on the second axis 6 at which the point 4 is displayed. Hence, if the first and second angles are each equal to zero, then the point 4 is displayed on the origin 8, as shown in Figure 2. If the monitor 1 is manipulated such that the current value of the roll angle exceeds the reference roll angle, then the point 4 moves towards the right hand side of the display 3. If the monitor 1 is manipulated such that the current value of the roll angle is less than the reference roll angle, then the point 4 moves towards the left hand side of the display 3. if the monitor 1 is manipulated such that the current value of the pitch angle exceeds the reference pitch angle, then the point 4 moves towards the upper side of the display 3. If the monitor 1 is manipulated such that current value of the pitch angle is less than the reference pitch angle, then the point 4 moves towards the lower side of the display 3.

In another embodiment there is a non-linear relationship between the values of the first and second angles and the position at which the point 4 is displayed. This nonlinear relationship may be used to depict the position of the point 4 with high sensitivity at positions close to the origin 8 and with progressively less sensitivity at positions spaced away from the origin.

The direction in which the line 7 extends from the origin 8 is determined with reference to the third angle. More particularly, the direction of the line 7 is selected such that the included angle between the line 7 and the second axis 6 is equal to the third angle. Therefore, if the current yaw angle of the monitor 1 is equal to the reference yaw angle, then the line 7 lies directly on the second axis 6, as shown in Figure 2. Hence, the second axis 6 is used as the reference indicium, however it will be appreciated that other indicia could be used as a reference indicium, such as the first axis 5 or another line or reference indicium that is displayed for this purpose.

The state of the point 4 and the line 7 as shown in Figure 2, in which the point 4 is substantially disposed on the origin 8 and the line 7 is substantially aligned with the second axis 6, indicates to the user that the current orientation of the monitor 1 corresponds to the reference orientation. Hence, if a user is intending to orient the implement into the reference orientation, then the user simply manipulates the monitor 1 in three dimensions until the state of the point 4 and the line 7 as shown in Figure 2 is displayed on the display 3.

A square 12 is depicted on the display 3 centered about the origin 8. The square 12 is sized such that the circle 11 fits neatly within it, as shown in Figure 2. This assists the user to confirm that the point 4 is positioned on the origin 8. Additionally, when the point 4 is positioned on the origin 8, the lines 9 and 10 overlie the inner portions of the first and second axes 5 and 6, which also assists the user to confirm that the point 4 is on the origin 8.

The display 3 also includes a display that is a set 13 of three numbers, which are the first, second and third angles. This provides additional useful information for the user, particularly if the desired orientation differs from the reference orientation. For example, a user may decide that the desired orientation should differ from, say, the reference yaw angle by a particular angle, say 5°, In this case, the user would manipulate the monitor 1 until the numeric reading shows (0, 0, 5).

While a number of preferred embodiments have been described, it will be appreciated by persons skilled in the art that numerous variations and/or modifications may be made to the invention without departing from the scope of the invention as broadly described. The present embodiments are, therefore, to be considered in all respects as illustrative and not restrictive.

## Claims

1. An electronic orientation monitor (1) comprising:
an orientation sensing electronics configured for calibration when in a reference orientation and being responsive to manipulation of the monitor so as to calculate a difference between a current orientation of the monitor and the reference orientation; and
a display (3), wherein the orientation sensing electronics is configured to calculate first, second and third angles which together represent the difference between the current orientation of the monitor and the reference orientation; and wherein the display (3) is responsive to the first and second angles so as to display a point (4) positioned relative to first (5) and second (6) axes, **characterized in that** a coordinate of the position of the point (4) on the first axis (5) is determined with reference to the first angle and a coordinate of the position of the point (4) on the second axis (6) is determined with reference to the second angle, the display (3) being further responsive to the third angle so as to display a line (7) having a direction relative to the first (5) and second (6) axes such that a combination of the position of the point (4) and the direction of the line (7) is indicative to a user of the difference between the current orientation of the monitor and the reference orientation;
wherein the line (7) extends from an origin (8) of the first (5) and second axes (6); and
wherein a current orientation that corresponds to the reference orientation is indicated on the display (3) by the point (4) being disposed on the origin (8) of the first (5) and second (6) axes and the line (4) being aligned with a reference indicium.

2. An electronic orientation monitor (1) according to Claim 1, **characterised in that** the first, second and third angles are respectively associated with a three dimensional reference system.

3. An electronic orientation monitor (1) according to Claim 2, **characterised in that** the three dimensional reference system is defined with reference to a roll angle, a pitch angle and a yaw angle.

4. An electronic orientation monitor (1) according to Claim 3, **characterised in that** the monitor is configured during calibration to sense and store a reference roll angle, a reference pitch angle and a reference yaw angle.

5. An electronic orientation monitor (1) according to Claim 4, **characterised in that** the monitor is configured to calculate the first angle by sensing a current roll angle and comparing the reference roll angle to the current roll angle.

6. An electronic orientation monitor (1) according to Claim 4 or 5, **characterised in that** the monitor is configured to calculate the second angle by sensing a current pitch angle and comparing the reference pitch angle to the current pitch angle.

7. An electronic orientation monitor (1) according to any one of Claims 4 to 6, **characterised in that** the monitor is configured to calculate the third angle by sensing a current yaw angle and comparing the reference yaw angle to the current yaw angle.

8. An electronic orientation monitor (1) according to any one of Claims 1 to 7, **characterised in that** the direction of the line (4) is determined with reference to the third angle.

9. An electronic orientation monitor (1) according to any one of claims 1 to 8, **characterised in that** the reference indicium is a predetermined one of the first (5) or second (6) axes.

10. An electronic orientation monitor (1) according to any one of Claims 1 to 9, **characterised in that** the point (4) is indicated on the display (3) as the point of intersection of two lines (9, 10).

11. An electronic orientation monitor (1) according to any one of Claims 1 to 10, **characterised in that** the point (4) is indicated on the display by the centre of a circle (11).

12. An electronic orientation monitor (1) according to any one of Claims 1 to 11, **characterised in that** the display includes a numeric display of the first, second and third angles.

## Patentansprüche

1. Elektronischer Ausrichtung Monitor (1), umfassend:
eine Ausrichtungs-Erfassungselektronik, welche zu einer Kalibration eingerichtet ist, wenn sie sich in einer Referenzausrichtung befindet, und auf eine Manipulation des Monitors reagiert, um so eine Differenz zwischen einer momentanen Ausrichtung des Monitors und der Referenzausrichtung zu berechnen; und
eine Anzeige (3),
wobei die Ausrichtungs-Erfassungselektronik dazu eingerichtet ist, einen ersten, zweiten und dritten Winkel zu berechnen, welche zusammen die Differenz zwischen der momentanen Ausrichtung des Monitors und der Referenzausrichtung repräsentieren; und
wobei die Anzeige (3) auf den ersten und zweiten Winkel reagiert, um so einen Punkt (4) anzuzeigen, welcher relativ zu einer ersten (5) und zweiten (6) Achse positioniert ist, **dadurch gekennzeichnet, dass**
eine Koordinate der Position des Punkts (4) an der ersten Achse (5) unter Bezugnahme auf den ersten Winkel bestimmt wird und eine Koordinate der Position des Punkts (4) an der zweiten Achse (6) unter Bezugnahme auf den zweiten Winkel bestimmt wird, wobei die Anzeige (3) ferner auf den dritten Winkel reagiert, um so eine Linie (7) anzuzeigen, welche eine Richtung relativ zu der ersten (5) und zweiten (6) Achse aufweist, so dass eine Kombination der Position des Punkts (4) und der Richtung der Linie (7) einem Benutzer die Differenz zwischen der momentanen Ausrichtung des Monitors und der Referenzausrichtung anzeigt;
wobei die Linie (7) sich von einem Ursprung (8) der ersten (5) und zweiten Achse (6) erstreckt; und
wobei eine momentane Ausrichtung, welche der Referenzausrichtung entspricht, an der Anzeige (3) dadurch angezeigt wird, dass der Punkt (4) an dem Ursprung (8) der ersten (5) und zweiten (6) Achse angeordnet ist und die Linie (4) mit einem Referenzvermerk ausgerichtet ist.

2. Elektronischer Ausrichtung Monitor (1) nach Anspruch 1, **dadurch gekennzeichnet, dass** der erste, zweite und dritte Winkel jeweils einem dreidimensionalen Referenzsystem zugeordnet sind.

3. Elektronischer Ausrichtung Monitor (1) nach Anspruch 2, **dadurch gekennzeichnet, dass** das dreidimensionale Referenzsystem unter Bezugnahme auf einen Rollwinkel, einen Nickwinkel und einen Gierwinkel definiert ist.

4. Elektronischer Ausrichtung Monitor (1) nach Anspruch 3, **dadurch gekennzeichnet, dass** der Monitor dazu eingerichtet ist, während der Kalibration einen Referenz-Rollwinkel, einen Referenz-Nickwinkel und einen Referenz-Gierwinkel zu erfassen und zu speichern.

5. Elektronischer Ausrichtung Monitor (1) nach Anspruch 4, **dadurch gekennzeichnet, dass** der Monitor dazu eingerichtet ist, den ersten Winkel zu berechnen, indem ein momentaner Rollwinkel erfasst wird und der Referenz-Rollwinkel mit dem momentanen Rollwinkel verglichen wird.

6. Elektronischer Ausrichtung Monitor (1) nach Anspruch 4 oder 5, **dadurch gekennzeichnet, dass** der Monitor dazu eingerichtet ist, den zweiten Winkel zu berechnen, indem ein momentaner Nickwinkel erfasst wird und der Referenz-Nickwinkel mit dem momentanen Nickwinkel verglichen wird.

7. Elektronischer Ausrichtung Monitor (1) nach einem der Ansprüche 4 bis 6, **dadurch gekennzeichnet, dass** der Monitor dazu eingerichtet ist, den dritten Winkel zu berechnen, indem ein momentaner Gierwinkel berechnet wird und der Referenz-Gierwinkel mit dem momentanen Gierwinkel verglichen wird.

8. Elektronischer Ausrichtung Monitor (1) nach einem der Ansprüche 1 bis 7, **dadurch gekennzeichnet, dass** die Richtung der Linie (4) unter Bezugnahme auf den dritten Winkel bestimmt wird.

9. Elektronischer Ausrichtung Monitor (1) nach einem der Ansprüche 1 bis 8, **dadurch gekennzeichnet, dass** der Referenzvermerk eine vorbestimmte aus der ersten (5) oder zweiten (6) Achse ist.

10. Elektronischer Ausrichtung Monitor (1) nach einem der Ansprüche 1 bis 9, **dadurch gekennzeichnet, dass** der Punkt (4) an der Anzeige (3) als der Schnittpunkt von zwei Linien (9, 10) angezeigt wird.

11. Elektronischer Ausrichtung Monitor (1) nach einem der Ansprüche 1 bis 10, **dadurch gekennzeichnet, dass** der Punkt (4) an der Anzeige durch den Mittelpunkt eines Kreises (11) angezeigt wird.

12. Elektronischer Ausrichtung Monitor (1) nach einem der Ansprüche 1 bis 11, **dadurch gekennzeichnet, dass** die Anzeige eine numerische Anzeige des ersten, zweiten und dritten Winkels umfasst.

## Revendications

1. Moniteur d'orientation électronique (1) comprenant :
un circuit électronique de détection d'orientation conçu pour un étalonnage lorsqu'il se trouve dans une orientation de référence et réagissant à une manipulation du moniteur de manière à calculer une différence entre une orientation actuelle du moniteur et l'orientation de référence ; et
un dispositif d'affichage (3),
dans lequel le circuit électronique de détection d'orientation est conçu pour calculer des premier, deuxième et troisième angles qui représentent conjointement la différence entre l'orientation actuelle du moniteur et l'orientation de référence ; et
dans lequel le dispositif d'affichage (3) réagit aux premier et deuxième angles de manière à afficher un point (4) placé par rapport à des premier (5) et deuxième (6) axes,
**caractérisé en ce qu'**une coordonnée de la position du point (4) sur le premier axe (5) est déterminée par rapport au premier angle et une coordonnée de la position du point (4) sur le deuxième axe (6) est déterminée par rapport au deuxième angle, le dispositif d'affichage (3) réagissant en outre au troisième angle de manière à afficher une ligne (7) ayant une direction par rapport aux premier (5) et deuxième (6) axes de sorte qu'une combinaison de la position du point (4) et de la direction de la ligne (7) indique à un utilisateur la différence entre l'orientation actuelle du moniteur et
l'orientation de référence ;
dans lequel la ligne (7) s'étend à partir d'une origine (8) des premier (5) et deuxième (6) axes ; et
dans lequel une orientation actuelle qui correspond à l'orientation de référence est indiquée sur le dispositif d'affichage (3) par le fait que le point (4) se trouve sur l'origine (8) des premier (5) et deuxième (6) axes et que la ligne (4) est alignée avec un repère de référence.

2. Moniteur d'orientation électronique (1) selon la revendication 1, **caractérisé en ce que** les premier, deuxième et troisième angles sont associés respectivement à un système de référence tridimensionnel.

3. Moniteur d'orientation électronique (1) selon la revendication 2, **caractérisé en ce que** le système de référence tridimensionnel est défini par rapport à un angle de roulis, un angle de tangage et un angle de lacet.

4. Moniteur d'orientation électronique (1) selon la revendication 3, **caractérisé en ce que** le moniteur est conçu pendant l'étalonnage pour détecter et stocker un angle de roulis de référence, un angle de tangage de référence et un angle de lacet de référence.

5. Moniteur d'orientation électronique (1) selon la revendication 4, **caractérisé en ce que** le moniteur est conçu pour calculer le premier angle en détectant un angle de roulis actuel et en comparant l'angle de roulis de référence à l'angle de roulis actuel.

6. Moniteur d'orientation électronique (1) selon la revendication 4 ou 5, **caractérisé en ce que** le moniteur est conçu pour calculer le deuxième angle en détectant un angle de tangage actuel et en comparant l'angle de tangage de référence à l'angle de tangage actuel.

7. Moniteur d'orientation électronique (1) selon l'une quelconque des revendications 4 à 6, **caractérisé en ce que** le moniteur est conçu pour calculer le troisième angle en détectant un angle de lacet actuel et en comparant l'angle de lacet de référence à l'angle de lacet actuel.

8. Moniteur d'orientation électronique (1) selon l'une quelconque des revendications 1 à 7, **caractérisé en ce que** la direction de la ligne (4) est déterminée par rapport au troisième angle.

9. Moniteur d'orientation électronique (1) selon l'une quelconque des revendications 1 à 8, **caractérisé en ce que** le repère de référence est l'un prédéterminé parmi les premier (5) et deuxième (6) axes.

10. Moniteur d'orientation électronique (1) selon l'une quelconque des revendications 1 à 9, **caractérisé en ce que** le point (4) est indiqué sur le dispositif d'affichage (3) comme étant le point d'intersection de deux lignes (9, 10).

11. Moniteur d'orientation électronique (1) selon l'une quelconque des revendications 1 à 10, **caractérisé en ce que** le point (4) est indiqué sur le dispositif d'affichage par le centre d'un cercle (11).

12. Moniteur d'orientation électronique (1) selon l'une quelconque des revendications 1 à 11, **caractérisé en ce que** le dispositif d'affichage comprend un affichage numérique des premier, deuxième et troisième angles.
